Europäisches Patentamt

European Patent Office

Office européen des brevets

(19)

(11) EP 0 802 175 A1

(12) EUROPEAN PATENT APPLICATION

(43) Date of publication:
22.10.1997 Bulletin 1997/43

(51) Int. Cl.6: C07C 67/36, C07C 69/36

(21) Application number: 97106147.8

(22) Date of filing: 15.04.1997

(84) Designated Contracting States:
BE DE ES FR GB IT NL

(30) Priority: 16.04.1996 JP 94529/96
16.04.1996 JP 94530/96

(71) Applicant: UBE INDUSTRIES, LTD.
Ube-shi, Yamaguchi-ken 755 (JP)

(72) Inventors:
• Ohdan, Kyoji,
Ube Industries, Ltd.
Ube-shi, Yamaguchi (JP)

• Matsuzaki Tokuo,
Ube Industries, Ltd.
Ube-shi, Yamaguchi (JP)
• Hidaka, Mikio,
Ube Industries, Ltd.
Ube-shi, Yamaguchi (JP)

(74) Representative: Kraus, Walter, Dr. et al
Patentanwälte Kraus, Weisert & Partner
Thomas-Wimmer-Ring 15
80539 München (DE)

(54) Process for preparation of dialkyl oxalate

(57) A dialkyl oxalate is prepared by reacting carbon monoxide and an alkyl nitrite in a gaseous phase in the presence of a catalyst. The catalyst is a porous carrier having a specific surface area in the range of 0.05 to 90 $m^2/g$ and a mean pore diameter in the range of 500 to 5,000 angstroms on which particles of metal belonging to platinum group are deposited.

EP 0 802 175 A1

## Description

FIELD OF THE INVENTION

The present invention relates to an improved process for preparing a dialkyl oxalate from carbon monoxide and an alkyl nitrate in a gaseous phase in the presence of a catalyst.

BACKGROUND OF THE INVENTION

Dialkyl oxalates such as dimethyl oxalate are of value as intermediates for producing oxalic acid, glycolic acid, dyes and pharmaceutically active compounds.

United States Patent 4,229,591 discloses that a dialkyl oxalate is prepared by reacting carbon monoxide with an alkyl nitrite in a gaseous phase in the presence of a catalyst composed of a solid carrier and a metal belonging to platinum group. Examples of the carrier include active carbon, alumina, silica, diatomaceous earth, pumice, zeolite and molecular sieve.

United States Patent 4,410,722 and European Patent Application No. 056,993-A disclose a process for a dialkyl oxalate by reacting carbon monoxide with an alkyl nitrite in a vapor (i.e., gaseous) phase in the presence of an alumina and a platinum-group metal or its salt supported on the alumina, said alumina having a specific surface area of not more than 90 $m^2$/g.

SUMMARY OF THE INVENTION

It is a principal object of the present invention to provide a new catalyst which is favorably employable for the process for preparing a dialkyl oxalate from carbon monoxide and an alkyl nitrite.

Specifically, the present invention has an object to provide a new catalyst for the preparation of dialkyl oxalates which can produce the desired dialkyl oxalates in increased space time yield (STY).

The present invention resides in a process for the preparation of a dialkyl oxalate by reacting carbon monoxide and an alkyl nitrite in a gaseous phase in the presence of a catalyst which comprises a porous carrier having a specific surface area in the range of 0.05 to 90 $m^2$/g and a mean pore diameter (or mean pore size) in the range of 500 to 5,000 angstroms and particles of metal belonging to platinum group deposited on the carrier.

The specific surface area is a BET specific surface area, and the mean pore diameter can be determined by means of a mercury porosimeter.

Preferred embodiments of the invention are stated below:

1) The above-mentioned process wherein the mean pore diameter of the carrier is in the range of 1,000 to 3,000 angstroms.

2) The above-mentioned process wherein the porous carrier has a pore volume in the range of 0.3 to 0.6 mL/g. The pore volume can be determined by means of a mercury porosimeter.

3) The above-mentioned process wherein the specific surface area of the carrier is in the range of 1 to 50 $m^2$/g.

4) The above-mentioned process wherein the porous carrier comprises alumina.

5) The above-mentioned process, wherein the porous carrier comprises material having a spinel structure.

6) The above-mentioned process wherein the porous carrier comprises material having a spinel structure and being represented by the formula $AD_2O_4$ or $A'D_5O_8$ wherein A is Mg, Zn, Co, Ni, Cu or Fe; A' is Li; and D is Al, Fe, Cr, Mn or Ga.

7) The above-mentioned process wherein the porous carrier comprises a lithium-containing spinel having the formula of $LiAl_5O_8$ or $Li_xAl_5O_{(15+x)/2}$ wherein x is a number of 0.5 to 1.5.

8) The above-mentioned process wherein the porous carrier comprises a magnesium-containing spinel having the formula of $MgAl_2O_4$ or $Mg_yAl_2O_{(3+y)}$ wherein y is a number of 0.5 to 1.5.

9) The above-mentioned process wherein the particles of metal are deposited on the carrier in an amount of 0.1 to 10 weight % based on the amount of carrier.

10) The above-mentioned process wherein the particles of metal have a mean particle size in the range of 10 to 100 angstroms.

11) The above-mentioned process wherein the metal belonging to platinum group is palladium.

12) The above-mentioned process wherein the reaction is conducted at a temperature of 50 to 200°C and a pressure of 1 to 20 kg/$cm^2$.

13) The above-mentioned process wherein the alkyl nitrite is methyl nitrite.

DETAILED DESCRIPTION OF THE INVENTION

The catalyst employed in the process of the invention comprises a porous carrier having a specific surface area in the range of 0.05 to 90 $m^2$/g and a mean pore diameter in the range of 500 to 5,000 angstroms and particles of metal belonging to platinum group deposited on the carrier.

[Catalyst Carrier]

The porous catalyst carrier of the invention may comprise alumina such as $\alpha$-alumina or $\theta$-alumina or material having a spinel structure.

The alumina having the small specific surface area and the large mean pore diameter can be chosen from commercially available alumina.

The material having a spinel structure gives X-ray diffraction peaks which originate from a crystal structure of the compound having the formula of $AD_2O_4$ or $A'D_5O_8$ (in which A is a divalent metal element, A' is a monovalent metal element, and D is a trivalent metal element). The material having a spinel structure, however, may not have the above-mentioned formula and may have the formula of $AD_2O_4$ or $A'D_5O_8$ in which an atom or atoms corresponding to A, A', and D are missing in part. In other words, the material having a spinel structure can be any material which gives X-ray diffraction peaks to be assigned to the any one of spinel structures. Atoms for A are divalent atoms such as Mg, Zn, divalent Co, Ni, divalent Cu and divalent Fe. Atoms for A' are monovalent atoms such as Li. Atoms for D are trivalent atoms such as Al, trivalent Fe, trivalent Cr, trivalent Mn, and Ga.

Examples of the materials having the spinel structure of $AD_2O_4$ include $MgAl_2O_4$, $ZnAl_2O_4$, $CoAl_2O_4$, $NiAl_2O_4$, $FeAl_2O_4$, $MgFe_2O_4$, $ZnFe_2O_4$, $CoFe_2O_4$, $NiFe_2O_4$, $CuFe_2O_4$, $FeFe_2O_4$, $MgCr_2O_4$, and $MgMn_2O_4$. Preferred is $MgAl_2O_4$.

Examples of the materials having the spinel structure of $A'D_5O_8$ include $LiAl_5O_8$, $LiFe_5O_8$, and $LiGa_5O_8$. Preferred is $LiAl_5O_8$.

Examples of the material having the spinel structure of the formula of $AD_2O_4$ or $A'D_5O_8$ in which an atom or atoms corresponding to A, A', and D are missing in part include lithium atom-defective $Li_xAl_5O_{(15+x)/2}$, and Mg atom-defective $Mg_yAl_2O_{(3+y)}$ in which x and y are numbers in the range of 0.5 to 1.5.

The material which has any one of the spinel structures, the small specific surface area and the large mean pore diameter can be chosen from commercially available materials having spinel structures. The desired spinel material can be produced by the known method, for instance, the method described in "Ceramic Technology Handbook" (edited by Japan Ceramic Society, Chapter 1, p. 873, published by Gihodo Publishing).

Otherwise, the desired material of the spinel structure can be produced by drying a mixture of a solution of a salt of a metal corresponding to A or A' and a solution of a salt of a metal corresponding to D and firing the dried mixture, or by impregnating an oxide of a metal corresponding to D with a solution of a salt of a metal corresponding to A or A', drying the impregnated oxide and then firing the dried product. For instance, the desired material having the spinel structure in which A' is Li and D is Al can be prepared from lithium nitrate and alumina by the below-mentioned process.

In an aqueous lithium nitrate solution (0.1 to 5 mol/L) are placed alumina particles or alimina sol in such an amount to give an atomic ratio of 1/5 in terms of Li/Al, at a temperature in the range of 20 to 80°C. The aqueous mixture was dried in a rotary evaporator, and the resulting product is dried at 110°C for 10 hours, and fired or calcined at a temperature of not lower than 1,100°C, preferably in the range of 1,100 to 1,600°C, to give the material having a mean pore diameter of 500 angstroms or more.

The desired material having the spinel structure in which a portion of A, A' and B is missing can be produced in the same manner by adjusting the amounts of the starting compounds.

The catalyst carrier can be in any forms, for instance, molded products such as pellets, particles and powder. The molded products preferably have a size in the range of 0.5 to 10 mm; the particles preferably have a particle size in the range of 4 to 200 meshes; and the powder preferably has a particle size in the range of 20 to 200 $\mu$m.

[Platinum Group Metal To Be Deposited On Carrier]

On the porous catalyst carrier is deposited a salt of a platinum group metal and the deposited salt is reduced to give the desired particles of the corresponding platinum group metal.

Examples of the platinum group metals include palladium, platinum, iridium, ruthenium and rhodium. Examples of the salts are halides, nitrates, phosphates, and sulfates.

Examples of the platinum group metal salts include halides (e.g., palladium chloride, palladium bromide, platinum chloride and rhodium chloride), nitrates (e.g., palladium nitrate and platinum nitrate), phosphates (e.g., palladium phosphate and ruthenium phosphate), and carboxylates (e.g., palladium acetate and rhodium acetate). The palladium salts are preferred, and most preferred are palladium chloride and palladium acetate. The platinum group metal salts can be employed singly or in combination.

The platinum group metal salt can be a coordination compound such as a platinum group metal coordinated with a nitrogen-containing compound or a phosphorus-containing compound. Examples of the nitrogen-containing compounds are ammonia and amines. Examples of the phosphorus-containing compounds are phosphines and phosphites, and the phosphine is preferred. The coordination compound can be formed from a platinum group metal salt and a nitrogen-containing compound or a phosphorus-containing compound in the presence of a catalyst carrier.

The ammonia may be employed in the form of a commercially available aqueous ammonia solution.

The amines may be aliphatic amines (e.g., trimethylamine and triethylamine), heterocyclic base compounds (e.g., pyridine, methylpyridine, dipyridine, and hydropyridine), and aromatic amines (e.g., aniline and triphenylamine). The nitrogen-containing compound can be employed singly or in combination.

The phosphine may be a monovalent phosphine or a divalent phosphine. Examples of the monovalent phosphines include trialkylphosphines (e.g., trimethylphosphine and triethylphosphine), triarylphosphines (e.g., triphenylphosphine) and tris(2-methoxyohenyl)phosphine), and diarylphosphines (e.g., diphenylmethylphosphine). Examples of the divalent phosphines include 1,2-diphenylphosphinoethane and 1,4-bis-diphenylphosphinobutane.

Examples of the phosphites include trialkylphosphites (e.g., trimethylphosphite, triethylphosphite, and tri-n-butylphosphite), and triarylphosphites (e.g., tri-phenylphosphite).

The phosphines and phosphites can be employed singly or in combination.

The platinum group metal salt can be employed in an aqueous solution or in a solution of an organic solvent (e.g., methanol). The solution generally contains the platinum group metal salt at a concentration of 0.01 to 10 weight %. If the coordination compound is employed, the ammonia, amines, phosphines or phosphites can be used in an amount of 0.1 to 10 mol., preferably 1 to 6 mol., per one mole of the platinum group metal.

The deposition of the platinum group metal on the porous carrier can be by placing the carrier in the solution of a platinum group metal at a temperature of 0 to 90°C for 1 to 20 hours, and drying thus treated carrier. The platinum group metal salt generally deposited on the carrier in an amount of 0.1 to 10 weight %, preferably 0.2 to 5 weight %, in terms of the amount of the metal. The amount of a platinum group metal salt or a platinum group metal on the carrier can be determined by means of an inductive combination plasma spectroscopic apparatus (ICP).

The carrier having the platinum group metal salt thereon is generally separated from the solution, washed with water, and dried. The drying process can be performed at a temperature of 50 to 150°C, at any one of an atmospheric pressure, a reduced pressure of not lower than 100 torr, or a pressure of not higher than 10 kg/cm$^2$, for a period of 0.5 to 20 hours. The drying process can be done under any circumferential conditions, but may preferably be performed under a nitrogen atmosphere or other inert atmospheres or in air. If desired, the dried product can be treated with an aqueous diluted alkali solution and washed with water.

The porous carrier on which the platinum group metal salt is deposited is then subjected to reducing treatment. The reducing treatment can be performed by bringing the platinum group metal salt on the carrier into contact with a reducing agent. In the present invention, the particles of a platinum group metal deposited on the porous carrier in the above manner preferably have a mean particle size of 10 to 100 angstroms, more preferably 20 to 50 angstroms. The particle size can be determined by X-ray diffraction (XRD), transmission electron microscopy (TEM) or CO adsorption.

The reducing treatment (i.e., reduction) can be performed in a liquid phase or a gaseous phase. The reduction in a liquid phase can be performed by placing the dried carrier in an aqueous reducing agent solution containing 1 to 50 wt.%, preferably 2 to 30 wt.%, of a reducing agent, at a temperature of 0 to 100°C, preferably 10 to 100°C. Examples of the reducing agents are hydrazine, formic acid, and sodium formate. The reducing agent is preferably employed in an amount of 1 to 100 moles per one mole of the deposited platinum group metal (in terms of the amount of metal).

Thus reduced catalyst can be washed with water and dried at 50 to 150°C under inert conditions such as a nitrogen gas atmosphere, and preferably is brought into contact with a gaseous oxygen diluted with an inert gas or air at an ambient temperature, so that a portion of the deposited platinum group metal is oxidized.

The reduction in a gaseous phase can be done by placing the heat treated carrier having the platinum group metal salt thereon into a reaction vessel for the production of a dialkyl oxalate and charging into the vessel a reducing gas such as hydrogen or carbon monoxide. In this case, the preparation of a dialkyl oxalate can be performed following the reduction.

[Production of Dialkyl Oxalate]

The dialkyl oxalate can be produced by gaseous catalytic reaction between an alkyl nitrite and carbon monoxide.

Examples of the alkyl nitrites include nitrite esters of lower saturated aliphatic monohydric alcohols having 1 to 8 carbon atoms such as methyl nitrite, ethyl nitrite, n-(or iso)propyl nitrite, and n-(or iso, or sec.-)butyl nitrite. The alkyl nitrite preferably is a nitrite of alkyl having 1 to 4 carbon atoms. Most preferred is methyl nitrite. Carbon monoxide can be industrially available carbon monoxide, and there is no severe limitation on its purity.

The gaseous catalytic reaction can be performed by introducing a gaseous mixture comprising an alkyl nitrite, carbon monoxide and nitrogen gas into a vessel in which the catalyst is placed. The reaction can be done by bringing the gaseous mixture into contact with the catalyst at a temperature of 50 to 200°C, preferably 80 to 150°C, a pressure of 1

to 20 kg/cm$^2$, preferably 1 to 10 kg/cm$^2$, for a period of 0.1 to 20 seconds, preferably 0.2 to 10 seconds. The gaseous mixture preferably contains an alkyl nitrite of 1 to 35 vol.%, most preferably 3 to 30 vol.%, and carbon monoxide of 1 to 90 vol.%, most preferably 5 to 60 vol.%.

The catalyst bed may be a fixed bed, a fluid bed, or a moving bed. The fixed bed is preferred from the viewpoint of industrial production.

In the above-mentioned manner, a dialkyl oxalate having an alkyl group of 1 to 8 carbon atoms such as dimethyl oxalate, diethyl oxalate, dipropyl oxalate, or dibutyl oxalate can be produced with a high space time yield and a high selectivity. The produced dialkyl oxalate can be easily recovered from the reaction mixture by distillation.

The present invention is further described by the following examples.

Example 1

1) Production of catalyst carrier

In an aqueous lithium nitrate solution (concentration: 1.96 mol./L) was immersed 30 g of a commercially available alumina catalyst carrier (spherical, mean diameter: 2 mm, specific surface area: 193 m$^2$/g). The aqueous mixture was kept for one hour at 25°C and placed in a rotary evaporator for distilling water off. The residue was heated at 110°C for 10 hours in air for dryness to obtain an alumina body on which lithium nitrate was deposited. The alumina body carrying lithium nitrate thereon was heated at 350°C for one hour and fired at 1,300°C for 5 hours. Thus, a porous lithium aluminate (LiAl$_5$O$_8$) having Li/Al of 1/5 (atomic ratio) was obtained. The spinel structure was confirmed by an X-ray diffraction chart (X-ray source: Cu-K$\alpha$) of the obtained porous lithium aluminate. The atomic ratio (Li/Al) was determined by X-ray fluorescence. The BET specific surface area of the obtained porous lithium aluminate was 30 m$^2$/g. The mean pore diameter and pore volume determined by a mercury porosimeter were 1,160 angstroms and 0.505 mL/g, respectively.

2) Production of catalyst

In 34.2 weight parts of aqueous hydrochloric acid (0.93 wt.%) was dissolved 0.77 weight part of palladium chloride. In the solution was placed the above-obtained lithium aluminate spinel (LiAl$_5$O$_8$), and the mixture was stirred at room temperature for approximately 2 hours. The lithium aluminate spinel having the palladium chloride on its surface was recovered by repeating decantation and washing with water and dried at 110°C. The dried product was placed in aqueous sodium formate solution (7 wt.%), and the mixture was stirred at 90°C for approximately 4 hours for performing reduction. The reduced product was recovered by repeating decantation and washing with water, and dried at 200°C in a nitrogen gas stream. The dried product was cooled to room temperature, and then gradually brought into contact with air. Thus obtained catalyst comprised the lithium aluminate spinel and 0.5 wt.% of palladium metal (in the form of particles having a mean particle size 23 angstroms) deposited on the surface of the spinel.

3) Preparation of dimethyl oxalate

The above-obtained catalyst (6.6 mL) was placed in a stainless steel reaction tube (inner diameter 16 mm, length 250 mm) and then heated so that the formed catalyst layer reached 110°C. The reaction was carried out at 110°C for 6 hours by passing through the catalyst layer a gaseous mixture (carbon monoxide of 20 vol.%, methyl nitrite of 15 vol.%, and nitrogen of remaining portion) in a flow rate of 20 NL/hr (space velocity: 3,000 hr$^{-1}$) at a pressure of 4 kg/cm$^2$.

The gaseous mixture recovered from the exit of the reaction tube was analyzed by gas chromatography. The results are as follows:

| STY of dimethyl oxalate: | 521 g/L・hr |
|---|---|
| STY of dimethyl carbonate: | 19 g/L・hr |
| STY of methylal: | 1.5 g/L・hr |
| STY of methyl formate: | 1.6 g/L・hr |
| (STY: space time yield, methylal: formaldehyde dimethyl acetal) | |

Example 2

1) Production of catalyst carrier

The procedures of Example 1 were repeated except for firing the alumina body carrying lithium nitrate thereon was fired at 1,400°C. Thus obtained porous lithium aluminate spinel ($LiAl_5O_8$) had a BET specific surface area of 24.4 $m^2$/g. The mean pore diameter and pore volume determined by a mercury porosimeter were 2,638 angstroms and 0.476 mL/g, respectively.

2) Production of catalyst

The procedures of Example 1 were repeated except for using the above-obtained porous lithium aluminate spinel. Palladium metal was deposited on the spinel carrier was 0.5 wt.% in the form of particles having a mean particle diameter of 35 angstroms.

3) Preparation of dimethyl oxalate

The procedures of Example 1 were repeated except for using the above-obtained catalyst.
The results are set forth in Table 1.

Example 3

1) Production of catalyst carrier

The procedures of Example 1 were repeated except for using 48 mL of the aqueous lithium nitrate solution and firing the alumina body carrying lithium nitrate thereon was fired at 1,400°C. Thus obtained porous lithium aluminate spinel ($Li_{0.8}Al_5O_{7.9}$) had a BET specific surface area of 13.0 $m^2$/g. The mean pore diameter and pore volume determined by a mercury porosimeter were 1,550 angstroms and 0.486 mL/g, respectively.

2) Production of catalyst

The procedures of Example 1 were repeated except for using the above-obtained porous lithium aluminate spinel. Palladium metal was deposited on the spinel carrier was 0.5 wt.% in the form of particles having a mean particle diameter of 30 angstroms.

3) Preparation of dimethyl oxalate

The procedures of Example 1 were repeated except for using the above-obtained catalyst.
The results are set forth in Table 1.

Example 4

1) Production of catalyst carrier

The procedures of Example 1 were repeated except for using 36 mL of the aqueous lithium nitrate solution and firing the alumina body carrying lithium nitrate thereon was fired at 1,350°C. Thus obtained porous lithium aluminate spinel ($Li_{0.6}Al_5O_{7.8}$) had a BET specific surface area of 8.0 $m^2$/g. The mean pore diameter and pore volume determined by a mercury porosimeter were 1,350 angstroms and 0.496 mL/g, respectively.

2) Production of catalyst

The procedures of Example 1 were repeated except for using the above-obtained porous lithium aluminate spinel. Palladium metal was deposited on the spinel carrier was 0.6 wt.% in the form of particles having a mean particle diameter of 25 angstroms.

3) Preparation of dimethyl oxalate

The procedures of Example 1 were repeated except for using the above-obtained catalyst.
The results are set forth in Table 1.

Example 5

1) Production of catalyst carrier

The procedures of Example 1 were repeated except for employing 59 mL of an aqueous magnesium nitrate solution (5 mol/L) in place of the aqueous lithium nitrate solution. Thus obtained porous magnesium aluminate spinel ($MgAl_2O_4$) had a BET specific surface area of 10.5 $m^2$/g. The mean pore diameter and pore volume determined by a mercury porosimeter were 2,100 angstroms and 0.505 mL/g, respectively.

2) Production of catalyst

The procedures of Example 1 were repeated except for using the above-obtained porous magnesium aluminate spinel. Palladium metal was deposited on the spinel carrier was 0.5 wt.% in the form of particles having a mean particle diameter of 28 angstroms.

3) Preparation of dimethyl oxalate

The procedures of Example 1 were repeated except for using the above-obtained catalyst.
The results are set forth in Table 1.

Example 6

1) Production of catalyst

The procedures of Example 1 were repeated except for using porous alumina ($\alpha$-alumina, sperical, mean diameter: 3 mm) having a specific surface area of 7.9 mL/g, a mean pore diameter of 1,500 angstroms and a pore volume of 0.350 mL/g in place of the porous lithium aluminate spinel. Palladium metal was deposited on the spinel carrier was 0.5 wt.% in the form of particles having a mean particle diameter of 35 angstroms.

2) Preparation of dimethyl oxalate

The procedures of Example 1 were repeated except for using the above-obtained catalyst.
The results are set forth in Table 1.

Example 7

1) Production of catalyst

In 30 weight parts of an aqueous ammonia (5 wt.%) were dissolved 1.12 weight parts of palladium chloride. In the resulting solution were immersed 100 weight parts of alumina ($\alpha$-alumina, spherical, mean particle diameter: 3 mm, specific surface area: 4.9 $m^2$/g, mean pore diameter: 2,757 angstroms, pore volume: 0.324 mL/g), and the mixture was stirred at 50°C for approximately 2 hours. The alumina body carrying palladium chloride thereon was recovered, washed with water, and dried at 100°C for 5 hours in air and then at 150°C for 3 hours in nitrogen stream. The dried product was placed in a solution of 0.5 weight part of sodium hydroxide in 50 weight parts of water, and the mixture was stirred at 70°C for approximately 9 hours ---- alkali treatment. The product having been subjected to the alkali treatment was repeatedly washed with water until a neutral washing containing no detectable chloride ion was obtained. The well washed product was placed in a solution of 3 weight parts of hydrazine (85 wt.%) in 97 weight parts of water, and the mixture was stirred at 30°C for approximately 3 hours ----reducing treatment. Thus reduced product was recovered by repeatedly decantation and washing with water, and dried at room temperature in nitrogen stream. The dried product was then gradually brought into contact with air. Thus obtained catalyst comprised $\alpha$-alumina and 0.56 wt.% of palladium metal (in the form of particles having a mean particle size 26 angstroms) deposited on the surface of the $\alpha$-alumina.

2) Preparation of dimethyl oxalate

The procedures of Example 1 were repeated except for using the above-obtained catalyst.
The results are set forth in Table 1.

Example 8

1) Production of catalyst

In 30 weight parts of an aqueous pyridine solution (5 wt.%) were dissolved 1.00 weight parts of palladium chloride. In the resulting solution were immersed 100 weight parts of alumina ($\alpha$-alumina, spherical, mean particle diameter: 3 mm, specific surface area: 7.9 $m^2$/g, mean pore diameter: 1,500 angstroms, pore volume: 0.330 mL/g), and the mixture was stirred at 50°C for approximately 2 hours. The alumina body carrying palladium chloride thereon was recovered, washed with water, and dried at 100°C for 5 hours in air and then at 150°C for 3 hours in nitrogen stream. The dried product was placed in a solution of 0.5 weight part of sodium hydroxide in 50 weight parts of water, and the mixture was stirred at 70°C for approximately 9 hours ---- alkali treatment. The product having been subjected to the alkali treatment was repeatedly washed with water until a neutral washing containing no detectable chloride ion was obtained. The well washed product was placed in an aqueous solution of sodium formate (7.7 wt.%), and the mixture was stirred at 90°C for approximately 3 hours ---- reducing treatment. Thus reduced product was recovered by repeatedly decantation and washing with water, and dried at 200°C in nitrogen stream. The dried product was then gradually brought into contact with air. Thus obtained catalyst comprised $\alpha$-alumina and 0.50 wt.% of palladium metal (in the form of particles having a mean particle size 35 angstroms) deposited on the surface of the $\alpha$-alumina.

2) Preparation of dimethyl oxalate

The procedures of Example 1 were repeated except for using the above-obtained catalyst.
The results are set forth in Table 1.

Example 9

1) Production of catalyst carrier

In 60 mL of an aqueous lithium nitrate solution (1.96 mol/L) were immersed 30 g of a commercially available alumina catalyst carrier (spherical, mean diameter: 3 mm, specific surface area: 195 $m^2$/g). The aqueous mixture was kept for one hour at 25°C and placed in a rotary evaporator for distilling water off. The residue was heated at 110°C for 10 hours in air for dryness to obtain an alumina body on which lithium nitrate was deposited. The alumina body carrying lithium nitrate thereon was heated at 350°C for one hour and fired at 1,300°C for 5 hours. Thus, a porous lithium aluminate ($LiAl_5O_8$) was obtained. The spinel structure was confirmed by an X-ray diffraction chart (X-ray source: Cu-K$\alpha$) of the obtained porous lithium aluminate. The BET specific surface area of the obtained porous lithium aluminate spinel was 24.4 $m^2$/g. The mean pore diameter and pore volume determined by a mercury porosimeter were 2,638 angstroms and 0.480 mL/g, respectively.

2) Production of catalyst

The procedures of Example 7 were repeated except for replacing the $\alpha$-alumina with the above-obtained porous lithium aluminate spinel and reducing the catalyst carrier carrying the palladium chloride with sodium formate at 80°C. Thus obtained catalyst comprised a lithium aluminate spinel and 0.56 wt.% of palladium metal (in the form of particles having a mean particle size 23 angstroms) deposited on the surface of the spinel.

3) Preparation of dimethyl oxalate

The procedures of Example 1 were repeated except for using the above-obtained catalyst.
The results are set forth in Table 1.

Example 10

1) Production of catalyst carrier

The procedures of Example 9 were repeated except for 30 mL of the aqueous lithium nitrate solution. Thus, a porous lithium aluminate spinel ($Li_{0.5}Al_5O_{7.8}$, specific surface area: 10.9 $m^2$/g, mean pore diameter: 1,800 angstroms, pore volume: 0.490 mL/g) was obtained.

2) Production of catalyst

The procedures of Example 8 were repeated except for using the above-obtained porous lithium aluminate spinel and using 40 weight parts of an aqueous dipyridine solution (2 wt.%).

The obtained catalyst comprised a lithium aluminate spinel and 0.60 wt.% of palladium metal (in the form of particles having a mean particle size 21 angstroms) deposited on the surface of the spinel.

3) Preparation of dimethyl oxalate

The procedures of Example 1 were repeated except for using the above-obtained catalyst.
The results are set forth in Table 1.

Example 11

1) Production of catalyst carrier

The procedures of Example 9 were repeated except for 42 mL of the aqueous lithium nitrate solution Thus, a porous lithium aluminate spinel ($Li_{0.7}Al_5O_{7.9}$, specific surface area: 15.6 $m^2$/g, mean pore diameter: 2,000 angstroms, pore volume: 0.495 mL/g) was obtained.

2) Production of catalyst

The procedures of Example 8 were repeated except for using the above-obtained porous lithium aluminate spinel and using 40 weight parts of a methanol solution of trimethylphosphine (5 wt.%). The reduction was performed using hydrazine in the same manner as in Example 7.

The obtained catalyst comprised a lithium aluminate spinel and 0.50 wt.% of palladium metal (in the form of particles having a mean particle size 29 angstroms) deposited on the surface of the spinel.

3) Preparation of dimethyl oxalate

The procedures of Example 1 were repeated except for using the above-obtained catalyst.
The results are set forth in Table 1.

Example 12

1) Production of catalyst carrier

The procedures of Example 9 were repeated except for 59 mL of an aqueous magnesium nitrate solution (5 mol/L). Thus, a porous magnesium aluminate spinel ($MgAl_2O_4$, specific surface area: 10.5 $m^2$/g, mean pore diameter: 2,100 angstroms, pore volume: 0.340 mL/g) was obtained.

2) Production of catalyst

The procedures of Example 7 were repeated except for using the above-obtained porous magnesium aluminate spinel.

The obtained catalyst comprised a magnesium aluminate spinel and 0.60 wt.% of palladium metal (in the form of particles having a mean particle size 30 angstroms) deposited on the surface of the spinel.

3) Preparation of dimethyl oxalate

The procedures of Example 1 were repeated except for using the above-obtained catalyst.
The results are set forth in Table 1.

Comparison Example 1

1) Production of catalyst

The procedures of Example 1 were repeated except for using porous alumina ($\alpha$-alumina, spherical, mean diameter: 3 mm) having a specific surface area of 15 $m^2$/g, a mean pore diameter of 430 angstroms and a pore volume of 0.350 mL/g in place of the porous lithium aluminate spinel. Palladium metal was deposited on the spinel carrier was 0.5 wt.% in the form of particles having a particle size of 20 angstroms.

2) Preparation of dimethyl oxalate

The procedures of Example 1 were repeated except for using the above-obtained catalyst.
The results are set forth in Table 1.

Table 1

| Ex. | chemical formula | Carrier | | pore vol. | Space Time Yield (g/L • hr) | | | | Selectivity (%) |
|---|---|---|---|---|---|---|---|---|---|
| | | BET m$^2$/g | pore size | | DMO | DMC | ML | MF | |
| 1 | $LiAl_5O_8$ | 30 | 1,160 | 0.505 | 521 | 19 | 1.5 | 1.6 | 93.9 |
| 2 | $LiAl_5O_8$ | 24.4 | 2,638 | 0.476 | 550 | 16 | 1.4 | 1.3 | 95.1 |
| 3 | $Li_{0.8}Al_5O_{7.9}$ | 13.0 | 1,550 | 0.486 | 585 | 14 | 1.7 | 1.5 | 95.6 |
| 4 | $Li_{0.6}Al_5O_{7.8}$ | 8.0 | 1,350 | 0.496 | 575 | 13 | 1.8 | 1.6 | 95.6 |
| 5 | $MgAl_2O_4$ | 10.5 | 2,100 | 0.505 | 550 | 15 | 1.9 | 2.1 | 94.7 |
| 6 | $\alpha\text{-}Al_2O_3$ | 7.9 | 1,500 | 0.350 | 509 | 17 | 3.1 | 2.5 | 93.2 |
| 7 | $\alpha\text{-}Al_2O_3$ | 4.9 | 2,757 | 0.324 | 592 | 6.9 | 1.6 | 1.5 | 97.1 |
| 8 | $\alpha\text{-}Al_2O_3$ | 7.9 | 1,500 | 0.330 | 589 | 6.5 | 1.7 | 1.7 | 97.0 |
| 9 | $LiAl_5O_8$ | 24.4 | 2,638 | 0.480 | 603 | 7.5 | 1.1 | 1.0 | 97.5 |
| 10 | $Li_{0.5}Al_5O_{7.8}$ | 10.9 | 1,800 | 0.490 | 611 | 6.1 | 1.0 | 0.9 | 97.8 |
| 11 | $Li_{0.7}Al_5O_{7.9}$ | 15.6 | 2,000 | 0.495 | 585 | 7.6 | 1.2 | 1.2 | 97.2 |
| 12 | $MgAl_2O_4$ | 10.5 | 2,100 | 0.340 | 575 | 9.5 | 1.3 | 1.1 | 96.8 |
| Comparison 1 | $\alpha\text{-}Al_2O_3$ | 15 | 430 | 0.350 | 445 | 14 | 2.0 | 1.5 | 94.2 |

Remarks
BET: specific surface area (measured BET value)
pore diameter: mean pore diameter (angstrom)
pore vol.: pore volume (mL/g)
CO: carbon monoxide, DMO: dimethyl oxalate,
DMC: dimethyl carbonate, ML: methylal,
MF: methyl formate
Selectivity: selectivity to produce DMO determined by the following formula:
Selectivity(%)=[2xDMO]/[2xDMO+2xDMC+4xMF+2xML]x100  [wherein each of DMO, DMC, MF and ML means a molar amount of each product produced in the reaction]

**Claims**

1. A process for the preparation of a dialkyl oxalate by reacting carbon monoxide and an alkyl nitrite in a gaseous phase in the presence of a catalyst which comprises a porous carrier having a specific surface area in the range of 0.05 to 90 m$^2$/g and a mean pore diameter in the range of 500 to 5,000 angstroms and particles of metal belonging to platinum group deposited on the carrier.

2. The process of claim 1, wherein the mean pore diameter of the carrier is in the range of 1,000 to 3,000 angstroms.

3. The process of claim 1, wherein the porous carrier has a pore volume in the range of 0.3 to 0.6 mL/g.

4. The process of claim 1, wherein the specific surface area of the carrier is in the range of 1 to 50 $m^2/g$.

5. The process of claim 1, wherein the porous carrier comprises alumina.

6. The process of claim 1, wherein the porous carrier comprises material having a spinel structure.

7. The process of claim 1, wherein the porous carrier comprises material having a spinel structure and being represented by the formula $AD_2O_4$ or $A'D_5O_8$ wherein A is Mg, Zn, Co, Ni, Cu or Fe; A' is Li; and D is Al, Fe, Cr, Mn or Ga.

8. The process of claim 1, wherein the porous carrier comprises a lithium-containing spinel having the formula of $LiAl_5O_8$ or $Li_xAl_5O_{(15+x)/2}$ wherein x is a number of 0.5 to 1.5.

9. The process of claim 1, wherein the porous carrier comprises a magnesium-containing spinel having the formula of $MgAl_2O_4$ or $Mg_yAl_2O_{(3+y)}$ wherein y is a number of 0.5 to 1.5.

10. The process of claim 1, wherein the particles of metal are deposited on the carrier in an amount of 0.1 to 10 weight % based on the amount of carrier.

11. The process of claim 1, wherein the particles of metal have a mean particle size in the range of 10 to 100 angstroms.

12. The process of claim 1, wherein the metal belonging to platinum group is palladium.

13. The process of claim 1, wherein the reaction is conducted at a temperature of 50 to 200°C and a pressure of 1 to 20 $kg/cm^2$.

14. The process of claim 1, wherein the alkyl nitrite is methyl nitrite.

European Patent
Office

**EUROPEAN SEARCH REPORT**

Application Number

EP 97 10 6147

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 057 630 A (UNION CARBIDE CORPORATION) <br><br> * page 9, line 11 - line 28 * <br> * page 14, line 28 - page 15, line 22 * <br> * page 16, line 3 - line 27 * <br> * page 22; table 1 * <br> * page 24; claims * <br> --- | 1-5, 10-14 | C07C67/36 <br> C07C69/36 |
| X | DATABASE WPI <br> Week 8804 <br> Derwent Publications Ltd., London, GB; <br> AN 88-021965 <br> XP002033913 <br> & DD 249 261 A (VEB LEUNA-WERKE ULBRICHT W) , 2 September 1987 <br> * abstract * <br> ----- | 1-5,10, 12-14 | |
| | | | TECHNICAL FIELDS SEARCHED (Int.Cl.6) <br><br> C07C |

The present search report has been drawn up for all claims

| Place of search | Date of completion of the search | Examiner |
|---|---|---|
| THE HAGUE | 27 June 1997 | Kinzinger, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document